# EUROPEAN PATENT APPLICATION

(11) **EP 0 574 066 A2**
(43) Date of publication of application: **15.12.1993**
(21) Application number: 93201551.4
(22) Date of filing: 01.06.1993
(51) Int. Cl.: C12P 19/04, C12P 1/00

(54) **Method for the recovery of enzymes used in polysaccharide modification processes**

(30) Priority: 09.06.1992 EP 92201657
(71) Applicant: QUEST INTERNATIONAL B.V., 1411 GP Naarden (NL)
(72) Inventor: Kamperman, Arie, NL-1403 VT Bussum (NL); Laane, Nicolaas Charles, NL-1412 GS Naarden (NL); Schmedding, Diederik Johannes Maria, NL-1412 NV Naarden (NL)
(74) Representative: Kan, Jacob Hendrik, Dr.

(57) **Abstract**

The invention relates to a method for modifying polysaccharides by subjecting the polysaccharides - introduced in an aqueous medium - to the action of a modifying enzyme. At the end of the enzymatic reaction the modified polysaccharide is precipitated by the addition of
- either an organic solvent selected from the group consisting of C₁₋₄ alcohols, C₃₋₄ ketones, C₁₋₄ alkylacetates and C₂₋₄ ethers,
- or an inorganic water-soluble salt whereafter the modifying enzyme remained in the obtained liquor is recovered for reuse purposes.

## Description

The invention relates to a method for modifying polysaccharides by subjecting the polysaccharides - introduced in an aqueous medium - to the action of a modifying enzyme. More in particular, the invention relates to a method of modifying polysaccharides using an enzyme wherein a considerable amount of the enzyme can be recovered.

Generally, there are various ways in which an enzymatic modification of a (poly) saccharide can be carried out. A few methods for modifying guar are set out below. In case of subjecting a galactomannan to enzymatic modification a reduction of the α-D-galactopyranosyl side chains i.e. of the galactose content of the galactomannan is achieved, resulting in a product having altered properties like improved gel-forming properties. More in particular it is pointed at the galactomannan "guar" having a high galactose content of about 40 wt.%. On account of this high galactose content the gel-forming properties of guar are considered insufficient. For improving these properties it is known to subject guar to the action of the enzyme α-D-galactosidase for reducing the galactose content to about 10-30 wt.%. In this respect it is referred to H.S. Dugal & J.W. Swansen, IPPTA, Vol.11, no.1, 1974, pages 29-35, showing the principles of treating guar with an enzyme or mixture of enzymes to break the bond between the side chains and the mannan backbone. It is mentioned that the reaction is terminated by heating for 5 min. at 80°C. Such an inactivation of the enzyme involved is necessary for preventing the preparation of a worthless product which has no or hardly any α-D-galactopyranosyl side chains left. However, nothing is indicated about a recovery method of the enzyme involved which enzyme is rather precious.

In EP-A- 0,121,960 a process for improving the gelling properties of guar is described according to which a part of the galactose side units are removed from the mannan main chain (backbone) with the help of a specific α-D-galactosidase enzyme. Apparently this enzyme has no detectable β-mannanase activity as the β-mannanase enzyme does cleave the principal mannan backbone of the galactomannan. Breaking of the mannan backbone reduces the viscosity of the solution to a level which is unacceptable for specific but important industrial applications. Although suitable enzymes - which can be obtained from vegetable sources (e.g. from lucerne, fenugreek, coffee beans or guar seed) or from bacterial or fungal sources - are rather precious due to the need of purifying the crude enzyme preparations from β-mannanase, it is brought to the fore that at the end of the process the enzyme is simply deactivated e.g. by a heat treatment at 80 or 100°C.

Further in EP-A- 0,192,401 a method for modifying the gelling properties of o.a. guar is disclosed which method is characterized by subjecting the guar in aqueous solution to the enzyme α-galactomannan galactosidase i.e. a α-D-galactosidase until at least about 10% of the galactose content in the guar has been split off. The α-D-galactosidase is derived from a specific group of Penicillium species. However, at the end of the modification method of the galactomannan involved the enzyme in question is deactivated by heating the reaction mixture.

Unfortunately, in none of these methods the enzyme can be recovered conveniently.

Surprisingly, Applicant has found a specific method for performing the conversion of a polysaccharide as indicated above, resulting in a considerable recovery of the modifying enzyme from the polymeric endproduct, thereby enabling the reuse of the enzyme in further polysaccharide modification processes. More in particular the method according to the invention is characterized in that
(a) the modified polysaccharide is precipitated by the addition of
   - either an organic solvent selected from the group consisting of C₁₋₄ alcohols, C₃₋₄ ketones, C₁₋₄ alkylacetates and C₂₋₄ ethers,
   - or an inorganic water-soluble salt;
(b) the modifying enzyme remained in the obtained liquor (supernatant or filtrate) is recovered for reuse purposes; and
(c) - in appropriate cases- the converted, especially in case of degraded side chains (advantageously in monomeric form) can be recovered from the obtained liquor.

Concerning the above statement of invention it is brought to the fore that the unity of invention is lain in the particular property of the group of specific precipitating agents according to the invention, i.e. these agents are characterized by only precipitating the modified polysaccharide from the aqueous reaction liquor and not the used modifying enzyme, which remains dissolved in said liquor.

With respect to the above-given statement of invention it is referred to a number of references in the art indicating that enzymes can be recovered from the crude enzyme (protein) sources by
- either salting out with the salt ammonium sulphate
- or the addition of the organic solvent isopropanol resulting in a precipitate of the enzyme. However, such a prior art method is contrary to the method according to the invention as for instance the side chain degrading enzyme α-D-galactosidase remains dissolved in the supernatant, whereas only the processed poly-saccharide i.e. the converted galactomannan substrate is precipitated. Examples of above-meant references are
   - JP 1291795 TAKARA SHUZA KK: this reference relates to the recovery of the proline peptidase from the crude production system i.e. a cultured mixture containing the fruit body or mycelium of Basidiomycola by means of salting out the homogenized top part of the cultured mixture with ammonium sulphate,
   - JP 1238510 TAKARA SHUZA KK: this reference discloses a cosmetic material containing a stable proteolytic enzyme recovered from the crude production system i.e. fruit by means of salting out with ammonium sulphate,
   - SU 1110801 VOKON TECH. INST.: this reference describes the recovery of a milk-coagulating proteinase from the crude production system i.e. Rhizopus pygmaues P1-8 by a.o. an aqueous extraction and a precipitation of the enzyme with ammonium sulphate,
   - SU 908796 MOSC FOOD IND. TECH.: this reference refers to the recovery of glucoamylase, prepared from the crude production system i.e. a microbial culture medium by salting out with ammonium sulphate.

Further to the above it is referred to the prior art indicating that enzymes may be separated with organic solvents like isopropylalcohol (= isopropanol) and acetone. For instance, US-A- 4,083,960, column 2, lines 1-15 discloses the precipitation of the enzyme β-galactosidase present in a toluene extract of cells of microorganisms with the help of the organic solvents acetone and isopropylalcohol.

Referring to step (c) of the method according to the invention it is brought forward that a separation of the modifying enzyme from the converted, in particular degraded side chains of the polysaccharide, both present in the liquor obtained after step (a), is particularly interesting in case of galactose as side chain or side chain component. In this way a kosher galactose can be prepared which in its turn can be used for the production of kosher foodproducts. Such a separation can be performed in a way known to a skilled man in the art, for instance by ultrafiltration.

Concerning the method of the invention it is brought to the fore that the definition of organic solvent encompasses as C₁₋₄ alcohols, methanol, ethanol, propanol, isopropanol, butan-1-ol and butan-2-ol, as C₃₋₄ ketones acetone, as C₁₋₄ alkylacetates, methylacetate, ethylacetate, propylacetate and butylacetate and as C₂₋₄ ethers diethylether. Preferably the solvents ethanol, methanol and most preferably isopropanol are applied. The amount of organic solvent necessary for stopping the enzymatic reaction by the precipitation of the obtained polysaccharide product is about 30-60 % w/w. Concerning the stability of the side chain degrading enzyme α-D-galactosidase it is brought to the fore that if isopropanol is used as the precipitating agent for the galactomannan, the obtained α-D-galactosidase containing medium is preferably kept at a temperature in the range of 0-10°C.

As indicated above the organic solvent used for precipitating the obtained polysaccharide product may be replaced by an inorganic water-soluble salt. More in particular such a salt may be selected from the group consisting of ammonium sulphate (NH₄)₂SO₄, magnesium sulphate (MgSO₄), sodium chloride (NaCl), sodium acetate (NaAc), sodium sulphate (Na₂SO₄) and sodium borate (Na₃BO₃). Preferably ammonium sulphate is used as the salt in the salting out procedure for the obtained polysaccharide product. The amount used may depend on the salt applied but is preferably in the range of 15-30 % w/w.

Representatives of polysaccharides, applicable to the method of the invention are galactomannans having a mannan backbone and α-D-galactopyranosyl side chains. Other representatives are pectin, amylopectin, arabinan, microbial polysaccharides (e.g. xanthan), kappa-carrageenan (3,6 anhydro forming enzyme; conversion to λ carrageenan), etc.

Examples of above-defined galactomannans are guar (obtainable from Cyanaposis tetragonolibus), lucerne (Medicago sativa) and fenugreek (Trigonellafoenum graecum) and locust bean gum (obtain-able from Ceratonia siligua). Further examples of galactomannans are reported in Adv. Carbohydr. Chem. Biochem. 35 (1978), 341 ff. These galactomannans contain between about 20 and 50 % w/w galactose. On account of this different galactose content the above mentioned galactomannans have different properties. For instance, locust bean gum having a galactose content of about 21 % w/w galactose can form complexes, observed as gels with other polysaccharides like xanthan or kappa-carrageenan, whereas guar having a galactose content of about 40 % w/w cannot. In view of the rather low cost price and great availability of guar which may be of low quality since the (soluble) product may easily be purified from contaminants, the method according to the invention is particularly suitable to guar. In view of the large amount of guar to be treated a recovery of the applied α-D-galactosidase is considered of economical importance.

As already indicated above the enzyme α-D-galactosidase may be extracted from vegetable and microbial sources. However, apart from the relatively high cost price (see EP-A-0,192,401, page 1, lines 25-27) the obtained α-D-galactosidase extracts generally also show some β-mannanase activity. For that reason use may be made of the α-D-galactosidase-product according to EP-A- 0,255,153 produced by genetically modified microorganisms. Further the added amount of enzyme may vary within wide limits but is preferably in the range of 10-60 Units per gram of galactomannan (determined according to the standard enzyme assay with para-nitrophenol-galactose as chromogenic substrate; see Example 4.1 of EP-A- 0,255,153).

The decrease of the galactose content in the galactomannans treated according to the invention is dependent on the use of the end product. Generally a reduced galactose content in the range of 10-30 % w/w, preferably 15-20 % w/w is considered acceptable.

The treatment of the galactomannans according to the invention is preferably performed in an aqueous medium containing 0.1-3 % w/w of the galactomannan, preferably guar. The reaction temperature may vary within wide limits but is preferably in the range of 15-40°C. The reaction time mainly depends on the required conversion rate of the starting material but is generally in the range of 1-10 hours.

For the sake of completeness it is remarked that specific examples of products gelated with galactomannans treated according to the invention are food and feed products, for example soft drinks, bakery jelly and instant chocolate pudding, see Food Technol. 27 (1973), page 26 ff.

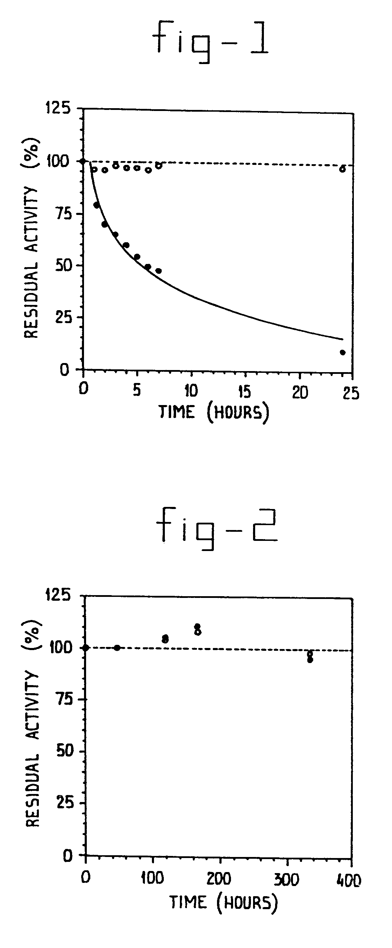

When the obtained enzyme solution is not reused instantaneously, the solution can best be kept at a low temperature to maintain enzyme activity at a sufficiently high level. This is exemplified by figs. 1 and 2 for guar modification.

### LEGENDA

- Fig. 1:: is a schematic illustration of the stability data of α-D-galactosidase in 40% isopropanol/water extracts obtained in Example I; the open symbols: T=4°C; and the closed symbols: T=20°C.
- Fig. 2:: is a schematic illustration of the stability data of α-D-galactosidase in 18% ammonium sulphate/water extracts obtained in Example II; the open symbols: T=4°C; and the closed symbols: T=20°C.

The invention is illustrated by means of the examples below; these examples may not be interpreted in a restrictive way.

Examples I and II describe a basic process according to the invention. Herein, guar is used as a substrate and α-D-galactosidase is used as the modifying enzyme. However, this method may be carried out with comparable results using the following substrate-enzyme combinations.

| substrate: | enzyme: |
|---|---|
| galactomannan guar | galactose-oxidase |
| pectin (methoxylated polygalacturonic acid) | pectin methyl-esterase |
| amylopectin | pullulanase |
| branched arabinan | arabinofuranosidase |
| starch | glucosyltransferase |
| alginate (a block polymer of guluronic and mannuronic acid) | mannuronan-C₅ epimerase |

### Example I

A homogeneous solution of guar in water was made by mixing 10 g of guar powder (Filgel P9155; as marketed by Quest International, Naarden, the Netherlands) with 15 ml of isopropanol. This suspension was added to 1 liter of a 10 mM sodium acetate (NaAc) buffer (pH = 4.5) and the obtained mixture was stirred continuously. In order to obtain a guar solution which has reached its full viscosity, the solution was heated at 80°C for 15 minutes.

After cooling the guar solution to 40°C the volume was adjusted for the evaporation (up to 1 liter) whereafter 30 units of α-D-galactosidase (derived from Saccharomyces cerevisiae described in EP-A- 0,255,153) were added per gram of guar (i.e. a total amount of 300 units of enzyme). After an incubation time of 3 hours the enzymatic reaction was stopped by adding isopropanol to the solution up to an amount of 40 % w/w. The obtained precipitate was filtered on a 200 mesh nylon screen (75 µm) and dried in an oven drier. The resulting fibrous material was milled for meeting powder specification of (the modified) guar.

The α-D-galactosidase activity in the obtained filtrate which also contained ≧ 90% of the freed galactose was determined by means of a standard enzyme assay with PNPG (para-nitrophenol-galactose) as chromogenic substrate (see Example 4.1 of EP-A- 0.255.153). The determined value was about 70% (= ± 200 U), based on the initially added activity. In this respect it is stated that the enzyme activity in the guar solution i.e. the incubation solution itself was reduced after the three hours incubation time to less than 80% of the initial enzyme activity, i.e. only a minor amount of the enzyme is co-precipitated with the modified guar.

The above-recovered α-D-galactosidase activity may be reused for further enzymatic treatments of guar. However, it is expected that the enzyme stability diminishes at elevated temperatures. For that reason enzyme stability tests in 40% isopropanol/water extracts have been performed showing that enzyme stability is sufficient at low temperatures, for instance 4°C, whereas the enzyme stability at higher temperatures like 20°C is considered insufficient for extended storage periods (≧ 5 hours). The tests were performed with 40% isopropanol/water-extracts having a α-D-galactosidase content of 3 % w/w. At specific time intervals (i.e. at 0, 1, 2, 3, 4, 5 and 24 hours) the enzyme activity was determined by the above-identified standard enzyme assay with PNPG. The results of the stability tests are illustrated in Fig. 1.

### Example II

The method as described in Example 1 has been carried out with the proviso that after an incubation period of one hour the enzymatic reaction was stopped by adding ammonium sulphate up to an amount of 18 % w/w to the solution.

The α-D-galactosidase activity in the obtained filtrate was determined by means of the standard assay defined in Example I. The determined value of the enzyme activity was about 70%, based on the initially added activity. In this respect it is pointed at the fact that during the incubation stage the initial enzyme activity of the guar solution was already reduced to less than 80%.

The stability of the above-recovered α-D-galactosidase, which may be reused for further enzymatic guar treatments, was investigated in the way defined in Example I. The samples were 18% ammonium sulphate/water extracts having a α-D-galactosidase content of 3 % w/w. The results of the stability tests are illustrated in Fig. 2 showing the good storage properties of the samples at both 4°C and 20°C.

## Claims

1. A method for performing the modification of polysaccharides by subjecting the polysaccharides introduced in an aqueous medium to the action of modifying enzyme, characterized in that
(a) the modified polysaccharide is precipitated by the addition of
- either an organic solvent (selected from the group consisting of C₁₋₄ alcohols C₃₋₄ ketones, C₁₋₄ alkylacetates and C₂₋₄ ethers),
- or an inorganic water-soluble salt;
(b) the modifying enzyme, remained in the obtained liquor, is recovered for reuse purposes; and
(c) -if desirable- the reaction product present in the obtained liquor is recovered.

2. The method according to Claim 1, characterized in that the organic solvent is selected from the group consisting of isopropanol, methanol, ethanol and acetone.

3. The method according to Claim 2, characterized in that the organic solvent is isopropanol.

4. The method according to Claims 1-3, characterized in that the organic solvent is used in an amount of 30-60 % w/w.

5. The method according to Claim 1, characterized in that the inorganic water-soluble salt is selected from the group consisting of (NH₄)₂SO₄, MgSO₄, NaCl, NaAc, Na₂SO₄ and Na₃BO₃.

6. The method according to Claim 5, characterized in that (NH₄)₂SO₄ is used as the inorganic water-soluble salt.

7. The method according to Claims 5 or 6, characterized in that salt is used in an amount of 15-30 % w/w.

8. The method according to any of Claims 1-7, characterized in that the polysaccharide is a galactomannan, pectin, amylopectin, arabinan, starch, alginate and kappa-carrageenan.

9. The method according to Claim 8, characterized in that the galactomannan is guar.

10. The method according to Claim 9, characterized in that the guar content in the aqueous medium is 0.1-3 wt.%.

11. The method according to Claims 9 or 10 characterized in that in case of the use of α-D-galactosidase as modifying enzyme, the obtained medium containing this enzyme is stored at a temperature in the range of 0-10°C.
